# NEUE EUROPÄISCHE PATENTSCHRIFT

(11) **EP 1 186 606 B2**
(45) Veröffentlichungstag und Bekanntmachung des Hinweises auf die Entscheidung über den Einspruch: **07.09.2011**
(45) Hinweis auf die Patenterteilung: 17.03.2004
(21) Anmeldenummer: 01127352.1
(22) Anmeldetag: 18.11.1996
(51) Int. Cl.: C07D 417/06, C07D 493/04, C07D 497/18, A61K 31/425, A01N 43/78, A61P 35/00

(54) **Epothilon-Derivate, ihre Herstellung und Verwendung**
Epothilone derivatives, their preparation and utilization
Derivés d'épothilone, leur procédé de production et utilisation

(30) Priorität: 17.11.1995 DE 19542986; 25.09.1996 DE 19639456
(43) Veröffentlichungstag der Anmeldung: 13.03.2002
(62) Teilanmeldung aus: 96939097.0
(73) Patentinhaber: Gesellschaft für Biotechnologische Forschung mbH (GBF), 38124 Braunschweig (DE)
(72) Erfinder: Höfle, Gerhard, Prof. Dr., 38124 Braunschweig (DE); Kiffe, Michael, Dr., 38124 Braunschweig (DE)
(74) Vertreter: Boeters, Hans Dietrich

(56) Entgegenhaltungen:
- WO-A-93/10121

## Beschreibung

Die vorliegende Erfindung betrifft allgemein Epothilonderivate und deren Verwendung zur Herstellung von Arzneimitteln. Insbesondere betrifft die vorliegende Erfindung die Herstellung der Epothilonderivate der nachfolgend dargestellten allgemeinen Formeln 1, 2 und 5 sowie deren Verwendung zur Herstellung von therapeutischen Mitteln und Mitteln für den Pflanzenschutz.

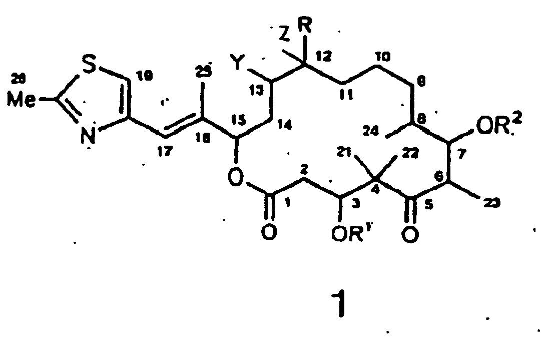

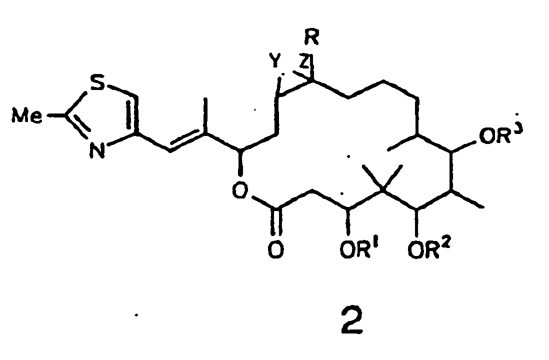

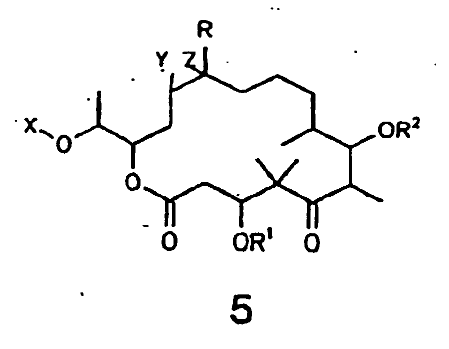

In den vorstehenden Formeln 1 bis Formel 7 bedeuten:
- R =: H, C₁₋₄-Alkyl;
- R¹, R², R³, R⁴, R⁵ =: H, C₁₋₆-Alkyl,
C₁₋₆-Acyl-Benzoyl,
C₁₋₄-Trialkylsilyl,
Benzyl,
Phenyl,
C₁₋₆-Alkoxy-,
C₆-Alkyl-, Hydroxy- und Halogen-substituiertes Benzyl bzw. Phenyl;
wobei es sich bei den in den Resten enthaltenen Alkyl- bzw. Acylgruppen um gradkettige oder verzweigte Reste handelt;
Y und Z eine der C-C-Bindungen einer C=C-Doppelbindung bilden.

In der **Formel 5** bedeutet X Wasserstoff, C₁₋₁₈-Alkyl, C₁₋₁₈-Acyl, Benzyl, Benzoyl und Cinnamoyl.

Zur Herstellung der erfindungsgemäßen Verbindungen kann man von Epothilon D ausgehen, wobei zur Derivatisierung auf die nachstehend für Epothilon A und B beschriebenen Derivatisierungsmethoden verwiesen werden kann. Für Epothilon A und B sei verwiesen auf DE-A-41 38 042.

Verbindungen gemäß der allgemeinen **Formel 2** sind aus Epothilon A und B sowie deren 3-O- und/oder 7-O-geschützten Derivaten durch Reduktion, z.B. mit NaBH₄ in Methanol erhältlich. Sind dabei 3-OH und/oder 7-OH reversibel geschützt, so können nach Acylierung oder Alkylierung und Entfernen der Schutzgruppen 5-O-monosubstituierte, 3,5- oder 5,7-O-disubstituierte Derivate der allgemeinen Formel 2 erhalten werden.

C-16-Ketone werden aus Epothilon A und B oder ihren 3-O- und/oder 7-O-geschützten Derivaten durch Ozonolyse und reduktive Aufarbeitung, z.B. mit Dimethylsulfid, erhalten.

Durch Reduktion der C-16-Ketogruppe, z.B. mit einem Aluminiumoder Borhydrid, sind die 16-Hydroxyderivate gemäß der allgemeinen **Formel 5** erhältlich. Diese können, wenn 3-OH und 7-OH mit entsprechenden Schutzgruppen versehen sind, selektiv acyliert oder alkyliert werden. Die Freisetzung der 3-OH- und 7-OH-Gruppen erfolgt z.B. bei O-Formyl durch NH₃/MeOH, bei O-p-Methoxybenzyl durch DDQ.

Erfindungsgemäß kann man die 12,13-Doppelbindung von Epothilon D epoxidieren, beispielsweise mit Dimethyldioxiran oder einer Persäure.

Die Erfindung betrifft ferner Mittel für den Pflanzenschutz in Landwirtschaft, Forstwirtschaft und/oder Gartenbau, bestehend aus einer oder mehreren der vorstehend aufgeführten Epothilonderivate bzw. bestehend aus einem oder mehreren der vorstehend aufgeführten Epothilonderivate neben einem oder mehreren üblichen Träger(n) und/oder Verdünnungsmittel(n).

Schließlich betrifft die Erfindung therapeutische Mittel, bestehend aus einer oder mehreren der vorstehend aufgeführten Verbindungen oder einer oder mehreren der vorstehend aufgeführten Verbindungen neben einem oder mehreren üblichen Träger(n) und/oder Verdünnungsmittel(n). Diese Mittel können insbesondere cytotoxische Aktivitäten zeigen und/oder Immunsuppression bewirken und/oder zur Bekämpfung maligner Tumore eingesetzt werden, wobei sie besonders bevorzugt als Cytostatika verwendbar sind.

Die Erfindung wird im folgenden durch die Beschreibung von einigen ausgewählten Ausführungsbeispielen näher erläutert und beschrieben.

### Beispiele

### Referenzbeispiel 1

### Verbindung 2a

100 mg (0.203 mmol) Epothilon A werden in 4 ml Tetrahydrofuran/1 M Phosphatpuffer pH 7 (1 : 1) gelöst und solange mit Natriumborhydrid (150 mg = 3.965 mmol) versetzt bis das Edukt laut Dünnschichtchromatogramm vollständig abreagiert ist. Anschließend wird mit 1 M Phosphatpuffer pH 7 verdünnt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes erfolgt durch Kieselchromatographie (Laufmittel: Dichlormethan/Aceton, 95 : 5 - grad - nach Dichlormethan/Aceton, 85 : 15).
- Ausbeute:: (20 %)
- R_{f} (Dichlormethan/Aceton, 75 : 25):: 0.27
- IR (Film):: ny = 3413 (s, b, Sch), 2965 (vs, Sch), 1734 (vs), 1458 (m, b, Sch), 1383 (m, Sch), 1264 (s, b, Sch), 1184 (m, b, Sch), 1059 (s, Sch), 966 (s), 885 (w), 737 (m) cm⁻¹
- MS (20/70 eV) :: m/e (%) = 495 (6 [M⁺]), 477 (8), 452 (12), 394 (9), 364 (16), 306 (49) , 194 (19), 178 (35), 164 (100), 140 (40), 83 (21), 55 (27).
- Hochauflösung:: C₂₆H₄₁O₆NS ber.: 495.2655 für [M⁺]
gef.: 495.2623

### Verbindung 3a-d (a-d sind Stereoisomere)

100 mg (0.203 mmol) Epothilon werden in 3 ml Pyridin gelöst, mit 50 µl (0.686 mmol) Thionylchlorid versetzt und 15 Minuten bei Raumtemperatur gerührt. Anschließend wird mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes und Trennung der vier Stereoisomeren 3a-d erfolgt mit Hilfe der präparativen Schichtchromatographie (Laufmittel: Toluol/Methanol, 90 : 10).

### Verbindung 3a

- Ausbeute:: 4 mg (12 %)
- R_{f} (Toluol/Methanol, 90 : 10):: 0.50
- IR (Film):: ny = 2961 (m, b, Sch), 1742 (vs), 1701 (vs), 1465 (m, Sch), 1389 (m, Sch), 1238 (s, Sch), 1210 (vs, Sch), 1011 (s, Sch), 957 (s, b, Sch), 808 (m, Sch), 768 (s, Sch) cm⁻¹

### Referenzbeispiel 2

### Verbindung 4a

10 mg (0.020 mmol) Epothilon A werden in 2 ml Dichlormethan gelöst, auf -70 °C abgekühlt und anschließend 5 Minuten mit Ozon bis zur schwachen Blaufärbung behandelt. Das resultierende Reaktionsgemisch wird anschließend mit 0.5 ml Dimethylsulfid versetzt und auf Raumtemperatur erwärmt. Zur Aufarbeitung wird das Reaktionsgemisch vom Lösungsmittel befreit und schließlich durch präparative Schichtchromatographie (Laufmittel Dichlormethan/Aceton/Methanol, 85 : 10 : 5) gereinigt.
- Ausbeute:: 5 mg (64 %)
- R_{f} (Dichlormethan/Aceton/Methanol, 85 : 10 : 5):: 0.61
- IR (Film):: ny = 3468 (s, br, Sch), 2947 (s, br, Sch), 1734 (vs, Sch), 1458 (w), 1380 (w) , 1267 (w), 1157 (w) , 1080 (w), 982 (w) cm⁻¹.
- UV (Methanol) :: lambdaₘₐₓ (lg epsilon) = 202 (3.53) nm.
- MS (20/70 eV):: m/e (%) = 398 (2 [M⁺]), 380 (4), 267 (14), 249 (17), 211 (20), 193 (26), 171 (34), 139 (34), 111 (40), 96 (100), 71 (48) , 43 (50).
- Hochauflösung:: C₂₁H₃₄O₇ ber.: 398.2305 für [M⁺]
gef.: 398.2295

### Beispiel 1:

### Epothilon D als Ausgangsverbindungen

### A. Produktionsstamm und Kulturbedingungen entsprechend dem Epothilon Basispatent.

### B. Produktion mit DSM 6773

75 l Kultur werden wie im Basispatent beschrieben angezogen und zum Animpfen eines Produktionsfermenters mit 700 l Produktionsmedium aus 0.8 % Stärke, 0.2 % Glukose, 0.2 % Soyamehl, 0.2 % Hefeextrakt, 0.1 % CaCl₂ x 2H₂O, 0.1 % MgSO₄ x 7H₂O, 8 mg/l Fe-EDTA, pH = 7.4 und optional 15 l Adsorberharz Amberlite XAD-16 verwendet. Die Fermentation dauert 7 - 10 Tage bei 30 C, Belüftung mit 2 m³ Luft/h. Durch Regulierung der Drehzahl wird der pO₂ bei 30 % gehalten.

### C. Isolierung

Das Adsorberharz wird mit einem 0.7 m², 100 mesh Prozeßfilter von der Kultur abgetrennt und durch Waschen mit 3 Bettvolumen Wasser/Methanol 2:1 von polaren Begleitstoffen befreit. Durch Elution mit 4 Bettvolumen Methanol wird ein Rohextrakt gewonnen, der i. Vak. bis zum Auftreten der Wasserphase eingedampft wird. Diese wird dreimal mit dem gleichen Volumen Ethylacetat extrahiert. Eindampfen der organischen Phase ergibt 240 g Rohextrakt, der zwischen Methanol und Heptan verteilt wird, um lipophile Begleitstoffe abzutrennen. Aus der Methanolphase werden durch Eindampfen i. Vak. 180 g Raffinat gewonnen, das in drei Portionen über Sephadex LH-20 (Säule 20 x 100 cm, 20 ml/min Methanol) fraktioniert wird. Die Epothilone sind in der mit 240 - 300 min Retentionszeit eluierten Fraktion von insgesamt 72 g enthalten. Zur Trennung der Epothilone wird in drei Portionen an Lichrosorb RP-18 (15 µm, Säule 10 x 40 cm, Laufmittel 180 ml/min Methanol/Wasser 65:35) chromatographiert. Nach Epothilon A und B werden mit Rₜ = 90-95 min Epothilon C und 100-110 min Epothilon D eluiert und nach Eindampfen i. Vak. in einer Ausbeute von jeweils 0.3 g als farblose Öle gewonnen.

### D. Physikalische Eigenschaften

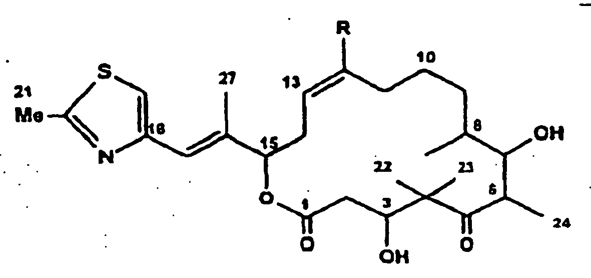

- Epothilon D: R = CH₃

### Epothilon D

C₂₇H₄₁NO₅S [491]
ESI-MS: (positiv Ionen): 492,5 für [M+H]⁺
1H und 13C siehe NMR-Tabelle
DC:R_{f} = 0,82
DC-Alufolie 60 F 254 Merck, Laufmittel: Dichlormethan/Methanol = 9:1
- Detektion:: UV-Löschung bei 254 nm. Ansprühen mit Vanillin-Schwefelsäure-Reagenz, blau-graue Anfärbung beim Erhitzen auf 120 °C.
HPLC:Rₜ = 15,3 min
Säule: Nucleosil 100 C-18 7µm, 125 x 4 mm
Laufmittel: Methanol/Wasser = 65:35
Fluß: 1ml/min
Detection: Diodenarray

**Tabelle:**

| ¹H-und ¹³C-NMR Daten von Epothilon D in [D₆]DMSO bei 300 MHz | | | |
|---|---|---|---|
| | Epothilon D | | |
| H-Atom | δ (ppm) | C-Atom | δ (ppm) |
| | | 1 | 170.1 |
| | | | |
| 2-Ha | 2.35 | 2 | 39.0 |
| 2-Hb | 2.38 | 3 | 70.8 |
| 3-H | 4.10 | 4 | 53.2 |
| 3-OH | 5.08 | 5 | 217.4 |
| 6-H | 3.11 | 6 | 44.4 |
| 7-H | 3.48 | 7 | 75.5 |
| 7-OH | 4.46 | 8 | 36.3 |
| 8-H | 1.29 | 9 | 29.9 |
| 9-Ha | 1.14 | 10 | 25.9 |
| 9-Hb | 1.38 | 11 | 31.8∗ |
| 10-Ha | 1.14∗ | 12 | 138.3 |
| 10-Hb | 1.35∗ | 13 | 120.3 |
| 11-Ha | 1.75 | 14 | 31.6∗ |
| 11-Hb | 2.10 | 15 | 76.6 |
| 12-H | | 16 | 137.2 |
| 13-H | 5.08 | 17 | 119.2 |
| 14-Ha | 2.30 | 18 | 152.1 |
| 14-Hb | 2.65 | 19 | 117.7 |
| 15-H | 5.29 | 20 | 164.3 |
| 17-H | 6.51 | 21 | 18.9 |
| 19-H | 7.35 | 22 | 19.7 |
| 21-H₃ | 2.65 | 23 | 22.5 |
| 22-H₃ | 0.90 | 24 | 16.4 |
| 23-H₃ | 1.19 | 25 | 18.4 |
| 24-H₃ | 1.07 | 26 | 22.9 |
| 25-H₃ | 0.91 | 27 | 14.1 |
| 26-H₃ | 1.63 | | |
| 27-H₃ | 2.11 | | |

| | | | |
|---|---|---|---|
| ∗ Zuordnung vertauschbar | | | |

### Referenzbeispiel 3

### Epothilon A und 12,13-Bisepi-epothilon A aus Epothilon C

50 mg Epothilon A werden in 1.5 ml Aceton gelöst und mit 1.5 ml einer 0.07 molaren Lösung von Dimethyldioxiran in Aceton versetzt. Nach 6 Stunden Stehen bei Raumtemperatur wird i. Vak. eingedampft und durch präparative HPLC an Kieselgel (Laufmittel: Methyl-tert.butylether/Petrolether/Methanol 33:66:1) getrennt.

Ausbeute:
25 mg Epothilon A, Rₜ = 3,5 min (analyt. HPLC, 7 µm, Säule 4 x 250 mm, Laufmittel s. o., Fluß 1.5 ml/min) und
20 mg 12,13-Bisepi-epothilon A, Rₜ = 3.7 min, ESI-MS (pos. Ionen)
   m/z = 494 [M+H]⁺
   ¹H-NMR in [D₄] Methanol, ausgewählte Signale: delta = 4.32
   (3-H), 3.79 (7-H), 3.06 (12-H), 3.16 (13-H), 5.54 (15-H), 6.69 (17-H), 1.20 (22-H), 1.45 (23-H). 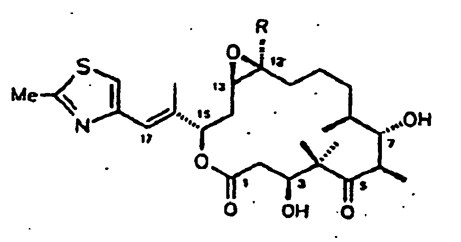
12,13-Bisepi-epothilon A R = H

## Patentansprüche

1. Epothilonderivat der Formel 1 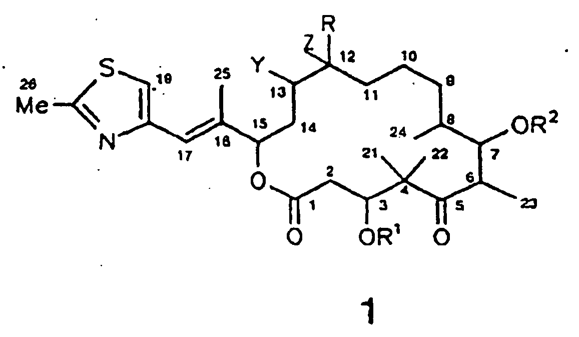 wobei
R = C₁-Alkyl;
R¹, R² = H und
Y und Z eine der C-C-Bindungen einer C=C-Doppelbindung bilden mit dem folgenden ¹H-NMR- und ¹³C-NMR-Spektrum für Epothilon D oder diese Verbindung,
wobei
R¹, R² = C₁₋₆-Alkyl, C₁₋₆-Acyl, Benzoyl, C₁₋₄-Trialkylsilyl" Benzyl, Phenyl; C₁₋₆-Alkoxy-, C₆-Alkyl, Hydroxy- und halogensubstituiertes Benzyl bzw. Phenyl; und es sich bei den in den Resten enthaltenen Alkyl- bzw. Acylgruppen um gradkettige oder verzweigte Reste handelt.
| ¹H-und ¹³C-NMR Daten von Epothilon D in [D₆] DMSO bei 300 MHz | | |
|---|---|---|
| Epothilon D | | |
| H-Atom δ | C-Atom | δ |
| (ppm) | | (ppm) |
| | 1 | 170.1 |
| 2.35 | 2 | 39.0 |
| 2.38 | 3 | 70.8 |
| 4.10 | 4 | 53.2 |
| 5.08 | 5 | 217.4 |
| 3.11 | 6 | 44.4 |
| 3.48 | 7 | 75.5 |
| 4.46 | 8 | 36.3 |
| 1.29 | 9 | 29.9 |
| 1.14 | 10 | 25.9 |
| 1.38 | 11 | 31.8 |
| 1.14 | 12 | 138.3 |
| 1.35 | 13 | 120.3 |
| 1.75 | 14 | 31.6 |
| 2.10 | 15 | 76.6 |
| | 16 | 137.2 |
| 5.08 | 17 | 119.2 |
| 2.30 | 18 | 152.1 |
| 2.65 | 19 | 117.7 |
| 5.29 | 20 | 164.3 |
| 6.51 | 21 | 18.9 |
| 7.35 | 22 | 19.7 |
| 2.65 | 23 | 22.5 |
| 0.90 | 24 | 16.4 |
| 1.19 | 25 | 18.4 |
| 1.07 | 26 | 22.9 |
| 0.91 | 27 | 14.1 |
| 1.63 | | |
| 2.11 | | |

2. Epothilonderivat der Formel 2 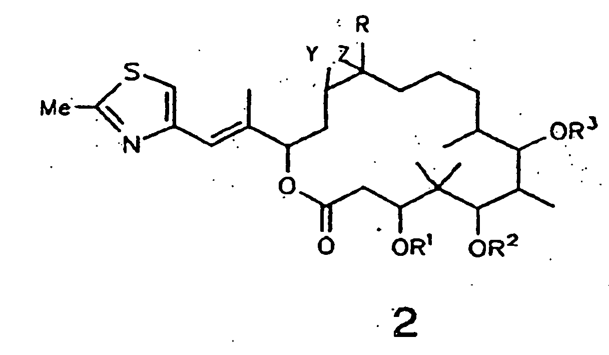 wobei
R = C₁-Alkyl;
R¹, R², R³ = H, C₁₋₆-Alkyl, C₁₋₆-Acyl, Benzoyl, C₁₋₄-Trialkylsilyl, Benzyl, Phenyl; C₁₋₆-Alkoxy-, C₆-Alkyl, Hydroxy- und halogensubstituiertes Benzyl bzw. Phenyl; es sich bei den in den Resten enthaltenen Alkyl- bzw. Acylgruppen um gradkettige oder verzweigte Reste handelt; und Y und Z die Bedeutung gemäß Anspruch 1 besitzen, **dadurch** herstellbar, dass man ausgehend von einem Epothilonderivat der Formel 1 gemäß Anspruch 1 eine Reduktion durchführt.

3. Epothilonderivat der Formel 5 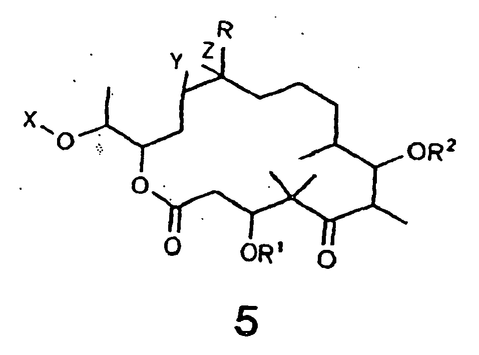 wobei
R = C₁-Alkyl
R¹, R² = H, C₁₋₆-Alkyl, C₁₋₆-Acyl, Benzoyl, C₁₋₄-Trialkylsilyl, Benzyl, Phenyl; C₁-₆-Alkoxy-, C₆-Alkyl, Hydroxy- und halogensubstituiertes Benzyl bzw. Phenyl; es sich bei den in den Resten enthaltenen Alkyl- bzw. Acylgruppen um gradkettige oder verzweigte Reste handelt, und
X Wasserstoff, C₁₋₁₈-Alkyl, C₁₋₁₈-Acyl, Benzyl, Benzoyl und Cinnamoyl bedeutet und
Y und Z die Bedeutungen gemäß Anspruch 1 besetzen, **dadurch** herstellbar, dass man ausgehend von einem Epothilon-Derivat der Formel 1 gemäß Anspruch 1 eine Ozonolyse-und reduktive Aufarbeitung durchführt und eine Reduktion der 16-Ketogruppe durchführt.

4. Verfahren zur Herstellung von Epothilon B und/oder 12,13-Bis-epi-epothilon B, **dadurch gekennzeichnet, dass** man Epothilon D epoxidiert, insbesondere mit Dimethyldioxiran oder einer Persäure.

5. Mittel für den Pflanzenschutz in der Landwirtschaft und Forstwirtschaft und/oder im Gartenbau, bestehend aus einer oder mehreren der Verbindungen gemäß einem der vorangehenden Ansprüche oder einer oder mehreren dieser Verbindungen neben einem oder mehreren üblichen Träger(n) und/oder Verdünnungsmittel(n).

6. Therapeutisches Mittel, insbesondere zum Einsatz als Cytostatikum, bestehend aus einer oder mehreren der Verbindungen nach einem oder mehreren der Ansprüche 1 bis 3 oder einer oder mehreren der Verbindungen nach einem oder mehreren der Ansprüche 1 bis 3 neben einem oder mehreren üblichen Träger(n) und/oder Verdünnungsmittel(n).

## Claims

1. Epothilone derivative of formula 1 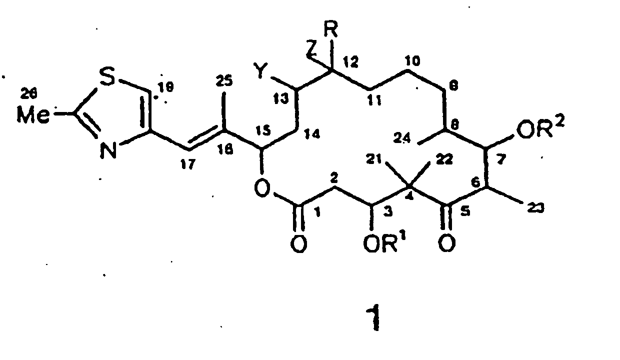 wherein
R = C₁-alkyl; R¹, R² = H and Y and Z together form a C-C bond of a C=C double bond,
having the following ¹H-NMR and ¹³C-NMR spectrum for epothilone D or said compound,
wherein
R¹, R² = C₁₋₆-alkyl, C₁₋₆-acyl, benzoyl, C₁₋₄-trialkylsilyl, benzyl, phenyl; benzyl or phenyl each substituted by C₁₋₆-alkoxy, C₆-alkyl, hydroxy or by halogen; and the alkyl and acyl groups contained in the radicals are straight-chain or branched radicals.
| ¹H- and ¹³C-NMR data of epothilone D in [D₆] DMSO at 300 MHz | | |
|---|---|---|
| epothilone D | | |
| H-atom δ | C-atom | δ |
| (ppm) | | (ppm) |
| | 1 | 170.1 |
| 2.35 | 2 | 39.0 |
| 2.38 | 3 | 70.8 |
| 4.10 | 4 | 53.2 |
| 5.08 | 5 | 217.4 |
| 3.11 | 6 | 44.4 |
| 3.48 | 7 | 75.5 |
| 4.46 | 8 | 36.3 |
| 1.29 | 9 | 29.9 |
| 1.14 | 10 | 25.9 |
| 1.38 | 11 | 31.8 |
| 1.14 | 12 | 138.3 |
| 1.35 | 13 | 120.3 |
| 1.75 | 14 | 31.6 |
| 2.10 | 15 | 76.6 |
| | 16 | 137.2 |
| 5.08 | 17 | 119.2 |
| 2.30 | 18 | 152.1 |
| 2.65 | 19 | 117.7 |
| 5.29 | 20 | 164.3 |
| 6.51 | 21 | 18.9 |
| 7.35 | 22 | 19.7 |
| 2.65 | 23 | 22.5 |
| 0.90 | 24 | 16.4 |
| 1.19 | 25 | 18.4 |
| 1.07 | 26 | 22.9 |
| 0.91 | 27 | 14.1 |
| 1.63 | | |
| 2.11 | | |

2. Epothilone derivative of formula 2 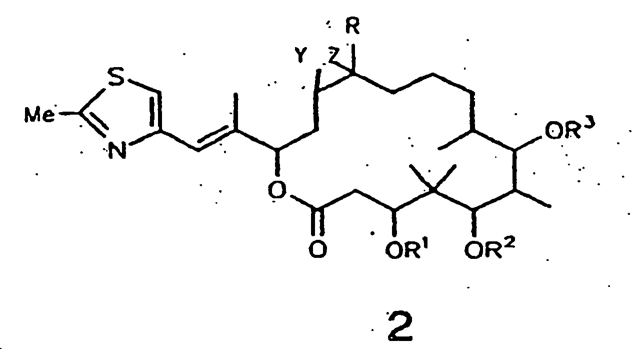 wherein
R = C₁-alkyl;
R¹, R², R³ = H, C₁₋₆-alkyl, C₁₋₆-acyl, benzoyl, C₁₋₄-trialkylsilyl, benzyl, phenyl, or benzyl or phenyl, each substituted by C₁₋₆-alkoxy, C₆-alkyl, hydroxy or by halogen; and the alkyl and acyl groups contained in the radicals are straight-chain or branched radicals; and
Y and Z have the meanings according to claim 1, wherein the epothilone derivative can be produced, starting from an epothilone derivative of formula 1 according to claim 1, by a reduction.

3. Epothilone derivative of formula 5 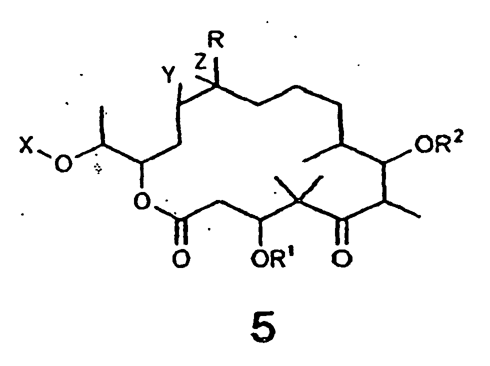 wherein
R = C₁-alkyl;
R¹, R² = H, C₁₋₆-alkyl, C₁₋₆-acyl, benzoyl, C₁₋₄-trialkylsilyl, benzyl, phenyl, or benzyl or phenyl each substituted by C₁₋₆-alkoxy, C₆-alkyl, hydroxy or by halogen; and the alkyl and acyl groups contained in the radicals are straight-chain or branched radicals; and
X represents hydrogen, C₁₋₁₈-alkyl, C₁₋₁₈-acyl, benzyl, benzoyl or cinnamoyl; and
Y and Z have the meanings according to claim 1, wherein the epothilone derivative can be produced, starting from an epothilone derivative of formula 1 according to claim 1, by ozonolysis and reductive treatment and a reduction of the 16-keto group.

4. Process for the preparation of epothilone B and/or 12,13-bis-epi-epothilone B, **characterized in that** epothilone D is epoxidised, especially with dimethyldioxirane or with a peracid.

5. Composition for plant protection in agriculture and forestry and/or in horticulture, consisting of one or more of the compounds according to any of the preceding claims or consisting of one or more of these compounds together with one or more common carrier(s) and/or diluents(s).

6. Therapeutic composition, especially for use as a cytostatic agent, consisting of one or more of the compounds according to one or more of claims 1 to 3 or consisting of one or more of the compounds according to one or more of claims 1 to 3 together with one or more common carrier(s) and/or diluent(s).

## Revendications

1. Dérivé d'épothilone, de formule 1 : 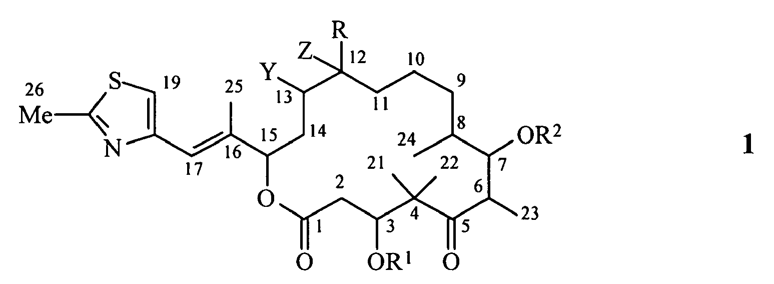 dans laquelle
R représente un groupe alkyle en C₁,
R¹ et R² représentent chacun un atome d'hydrogène,
et Y et Z constituent l'une des liaisons C-C d'une double liaison C=C,
lequel dérivé présente, pour l'épothilone D, les spectres de RMN-¹H et de RMN-¹³C indiqués ci-après,
ou composé de ce type, dans lequel
R¹ et R² représentent chacun un groupe alkyle en C₁₋₆, acyle en C₁₋₆, benzoyle, tri(alkyle en C₁₋₄)silyle, benzyle ou phényle, ou encore un groupe benzyle ou phényle portant un ou des substituant(s) alcoxy en C₁₋₆, alkyle en C₆, hydroxy ou halogéno, les fragments alkyle ou acyle figurant dans ces groupes étant linéaires ou ramifiés.
| Données de RMN-¹H et de RMN-¹³C pour l'épothilone D, dans du DMSO-D₆ et à 300 MHz | | |
|---|---|---|
| Epothilone D | | |
| H, δ (ppm) | C n° | δ (ppm) |
| | 1 | 170,1 |
| 2,35 | 2 | 39,0 |
| 2,38 | 3 | 70,8 |
| 4,10 | 4 | 53,2 |
| 5,08 | 5 | 217,4 |
| 3,11 | 6 | 44,4 |
| 3,48 | 7 | 75,5 |
| 4,46 | 8 | 36,3 |
| 1,29 | 9 | 29,9 |
| 1,14 | 10 | 25,9 |
| 1,38 | 11 | 31,8 |
| 1,14 | 12 | 138,3 |
| 1,35 | 13 | 120,3 |
| 1,75 | 14 | 31,6 |
| 2,10 | 15 | 76,6 |
| | 16 | 137,2 |
| 5,08 | 17 | 119,2 |
| 2,30 | 18 | 152,1 |
| 2,65 | 19 | 117,7 |
| 5,29 | 20 | 164,3 |
| 6,51 | 21 | 18,9 |
| 7,35 | 22 | 19,7 |
| 2,65 | 23 | 22,5 |
| 0,90 | 24 | 16,4 |
| 1,19 | 25 | 18,4 |
| 1,07 | 26 | 22,9 |
| 0,91 | 27 | 14,1 |
| 1,63 | | |
| 2,11 | | |

2. Dérivé d'épothilone, de formule 2 : 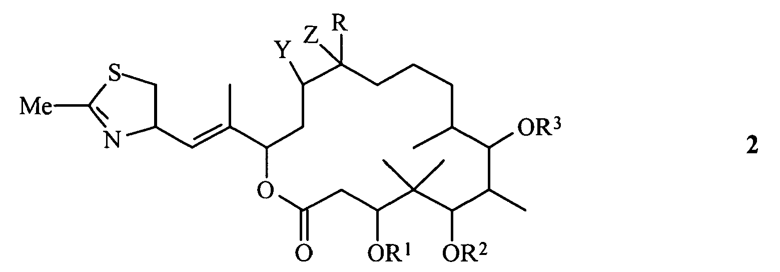 dans laquelle
R représente un groupe alkyle en C₁ ;
R¹, R² et R³ représentent chacun un atome d'hydrogène, un groupe alkyle en C₁₋₆, acyle en C₁₋₆, benzoyle, tri(alkyle en C₁₋₄)silyle, benzyle ou phényle, ou encore un groupe benzyle ou phényle portant un ou des substituant(s) alcoxy en C₁₋₆, alkyle en C₆, hydroxy ou halogéno, les fragments alkyle ou acyle figurant dans ces groupes étant linéaires ou ramifiés ;
et Y et Z ont la signification indiquée dans la revendication 1 ;
lequel dérivé peut être préparé par réduction, à partir d'un dérivé d'épothilone de formule 1, conforme à la revendication 1.

3. Dérivé d'épothilone, de formule 5 : 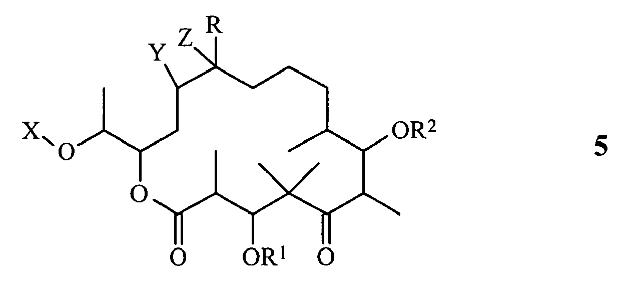 dans laquelle
R représente un groupe alkyle en C₁ ;
R¹ et R² représentent chacun un atome d'hydrogène, un groupe alkyle en C₁₋₆, acyle en C₁₋₆, benzoyle, tri(alkyle en C₁₋₄)silyle, benzyle ou phényle, ou encore un groupe benzyle ou phényle portant un ou des substituant(s) alcoxy en C₁₋₆, alkyle en C₆, hydroxy ou halogéno, les fragments alkyle ou acyle figurant dans ces groupes étant linéaires ou ramifiés ;
X représente un atome d'hydrogène ou un groupe alkyle en C₁₋₁₈, acyle en C₁₋₁₈, benzyle, benzoyle ou cinnamoyle ;
et Y et Z ont la signification indiquée dans la revendication 1 ;
lequel dérivé peut être préparé, à partir d'un dérivé d'épothilone de formule 1, conforme à la revendication 1, par ozonolyse et traitement réducteur, et réduction de la fonction cétone apparue en position 16.

4. Procédé de préparation d'épothilone B et/ou de 12,13-bis-épi-épothilone B, **caractérisé en ce qu'**on soumet de l'épothilone D à une époxydation, en particulier à l'aide de diméthyldioxirane ou d'un peracide.

5. Agent destiné à la protection de végétaux en agriculture, sylviculture et/ou horticulture, constitué de l'un ou de plusieurs des composés conformes à l'une des revendications précédentes, ou de l'un ou de plusieurs de ces composés en association avec un ou plusieurs véhicule(s) et/ou diluant(s) habituel(s).

6. Agent thérapeutique, destiné en particulier à être utilisé comme cytostatique, constitué de l'un ou de plusieurs des composés conformes à l'une ou plusieurs des revendications 1 à 3, ou constitué de l'un ou de plusieurs des composés conformes à l'une ou plusieurs des revendications 1 à 3 en association avec un ou plusieurs véhicule(s) et/ou diluant(s) habituel(s).
